# FASCICULE DE BREVET EUROPEEN

(11) **EP 0 992 212 B1**
(45) Date de publication et mention de la délivrance du brevet: **21.12.2005**
(21) Numéro de dépôt: 99430024.2
(22) Date de dépôt: 30.09.1999
(51) Int. Cl.: A61B 1/12, A61L 2/28, A61L 2/18

(54) **Paillasse de décontamination d'endoscopes souples avec traçabilité informatique**
Vorrichtung zur Dekontamination mit rechnergesteuerter Verfolgung flexibler Endoskope
Decontamination device having computer controlled tracing means for flexible endoscopes

(30) Priorité: 02.10.1998 FR 9812582
(43) Date de publication de la demande: 12.04.2000
(73) Titulaire: Medinorme S.à.r.l., 83500 La Seyne sur Mer (FR)
(72) Inventeur: Iffrig, Gérard, 83500 La Seyne sur Mer (FR)

(56) Documents cités:
- EP-A- 0 401 594
- EP-A- 0 709 056
- GB-A- 2 244 919
- US-A- 3 982 893
- US-A- 4 862 872

## Description

La présente invention a pour objet une paillasse de décontamination d'endoscopes souples.

Elle concerne le domaine industriel des équipements destinés au milieu hospitalier en général et a pour but d'apporter une réponse complète et fiable aux problèmes spécifiques liés à la décontamination et à la désinfection des fibroscopes, vidéo-endoscopes ou écho-endoscopes souples de tous types (gastroscopes, coloscopes, duodénoscopes, bronchoscopes, cytoscopes, etc.)

Afin de prévenir la transmission d'une infection exogène par apport de germes étrangers au patient lors d'une endoscopie, la législation en vigueur (circulaire DGS/DH n° 236 du 2 avril 1996, entre autres) prévoit plusieurs protocoles de traitement des endoscopes : un protocole long comportant toutes les étapes, qui doit être effectué après chaque acte d'endoscopie, un protocole court pour endoscope propre lorsque la dernière désinfection date de plus de douze heures (risque d'infection par l'air) et un protocole spécial si la dernière désinfection date de plus de seize heures (risque de formation de spores).

Le protocole long prévoit huit étapes :
1) Pré-traitement. Il est uniquement manuel et consiste en un essuyage avec des compresses pour éliminer les souillures visibles, aspiration des canaux et rinçage. Il s'effectue en général dans la zone d'examen.
2) Nettoyage. Il s'agit d'un lavage manuel avec brossage de l'extrémité, de tous les recoins et anfractuosités de l'endoscope et écouvillonnage soigneux de tous les canaux en vérifiant qu'ils ne sont pas obstrués.
3) Test d'étanchéité. Doit être pratiqué dès la première immersion par insufflation d'air ne dépassant pas 0,3 bar de pression dans l'endoscope pour gonfler la gaine distale. L'endoscope est immergé dans de l'eau et manipulé selon les recommandations du fabricant. Une observation attentive pendant le temps prescrit permet de vérifier l'absence de bulles et même de micro-bulles indiquant une fuite.
4) Décontamination. L'endoscope doit être trempé dans une solution détergente diluée dans l'eau du réseau. Cette solution doit passer dans toutes les lumières des canaux de l'endoscope et doit être renouvelée pour chaque usage.
5) Rinçage. Le rinçage a pour but d'éliminer les matières organiques résiduelles et toutes traces de détergent qui pourraient interférer avec le produit de désinfection utilisé ultérieurement. L'eau du réseau suffit pour ce rinçage qui doit être abondant, sous le robinet.
6) Désinfection. Doit se faire par un trempage d'une durée de 20 minutes dans un bac à fermeture hermétique par immersion et trempage dans une solution dans l'eau froide du réseau, d'un produit désinfectant bactéricide, fongicide et virucide sans activité détergente, tel que le glutaraldéhyde en solution à 2%.
7) Rinçage terminal. Il doit être abondant et pratiqué avec de l'eau filtrée sur membrane stérilisante de qualité prouvée pour l'endoscopie broncho-pulmonaire, ou de l'eau du réseau pour l'endoscopie digestive haute et basse non interventionnelle.
8) Séchage. Si l'endoscope n'est pas utilisé immédiatement, il doit être séché à l'air médical et stocké dans un endroit propre et sec à l'abri de toute source de contamination microbienne.

Les autres protocoles n'utilisent qu'une partie de ces étapes avec des paramètres de temps quelquefois plus longs.

A ce jour, le traitement des endoscopes se fait de façon souvent peu rigoureuse, sans aucun moyen de vérifier si les différentes étapes (et en particulier les étapes 2 à 8) ont bien été effectuées dans les conditions requises.

Le test d'étanchéité est généralement réalisé au moyen d'un testeur assurant la pression d'air par une poire à main munie d'un tuyau raccordé à l'endoscope, la pression à ne pas dépasser étant contrôlée visuellement à l'aide d'un manomètre.

Les paillasses actuelles utilisent généralement pour la décontamination des pompes basse pression dont la désinfection interne pose des problèmes ne permettant pas de garantir le résultat. Le rinçage s'effectue avec la même pompe, l'endoscope étant disposé sans être débranché dans un bac rempli d'eau du robinet.

Le rinçage se fait en circuit fermé, c'est à dire avec une eau de bac dans laquelle sont dilués les restes de produit décontaminant contenus malgré la purge dans les tuyaux, dans le corps de la pompe, dans les canaux et sur toute la partie extérieure de l'endoscope.

La désinfection est étroitement liée au problème des vapeurs toxiques du produit utilisé. Le produit désinfectant a une durée de vie dépendant à la fois du nombre d'appareils traités, son potentiel de neutralisation étant entamé par chaque traitement, et du temps passé dans le bac de désinfection. Les paillasses actuelles utilisent des pompes basse pression raccordées aux conduits de l'endoscope. Les vapeurs toxiques sont aspirées par des orifices situés en haut du bac, sous un couvercle de fermeture opaque, menant à des conduits remplis de charbon actif. L'air est ensuite sans autre traitement rejeté à l'extérieur de l'établissement. Le nettoyage de ces orifices est difficile. Le renouvellement des cartouches de charbon est malaisé et aléatoire, ce qui fait qu'elles sont souvent utilisées au delà de leur limite d'efficacité. Sur beaucoup de paillasses, le bac de désinfection n'est pas hermétique. L'endoscope est immergé dans la solution désinfectante et la marche de la pompe est contrôlée par une minuterie manuelle. La durée de traitement, pourtant essentielle puisque le virus des hépatites, les mycobactéries et les moisissures ne sont éliminées qu'au bout de 20 minutes, n'est pas rigoureusement assurée en cas d'erreur involontaire de manipulation de la minuterie ou en cas de volonté délibérée de gagner du temps. Le renouvellement du liquide de désinfection est effectué sans décompte exact du nombre de traitements effectués. Les informations des fabricants sur les limites d'efficacité de leur produit sont assez vagues pour ne pas permettre d'engager leur responsabilité.

Sur les paillasses actuelles, l'endoscope est, à la fin du temps de désinfection, transposé du bain dans un bac de rinçage final vide sans débrancher le raccordement à la pompe basse pression. L'endoscope se purge, comme pour le premier rinçage, par aspiration d'air. Le bac est ensuite rempli d'eau filtrée et la filtration s'effectue en circuit fermé avec l'eau du bac dans laquelle sont dilués les restes de produit désinfectant. Le séchage est effectué par une soufflette à main branchée par un flexible sur une prise d'air médical. La pression de l'air expulse l'humidité des anfractuosités de l'endoscope qui est ensuite essuyé avec des compresses stériles.

Le document US4862872 décrit un endoscope avec une puce d'identification, et un appareil de lavage avec un bac et avec un détecteur pour controler le rincage.

L'invention a pour but la mise au point d'une paillasse de décontamination d'endoscopes plus efficace et plus pratique que celles existant sur le marché, et équipée d'éléments permettant l'installation de modules optionnels, afin de s'adapter à l'évolution des règlements d'hygiène hospitalière et de prévention des maladies nosocomiales de plus en plus contraignants. Ces modules offrent la possibilité d'assurer d'une part une qualité microbiologique de plus en plus grande et d'autre part de répondre aux préoccupations grandissantes des hôpitaux et lieux de soins en matière de traçabilité (contrôles et justificatifs en cas de mise en cause), en matière de protection du personnel (problèmes cutanés et maux de tête dus au contact et aux vapeurs du glutaraldéhyde utilisé pour la désinfection) ainsi qu'en matière de protection de l'environnement (rejet des eaux souillées et rejet de l'air pollué de vapeurs nocives). Le dispositif revendiqué assure une traçabilité des opérations, une grande rigueur et donc une plus grande sûreté de la désinfection, ainsi qu'une meilleure qualité de travail en simplifiant les taches.

Le dispositif selon l'invention est constitué d'une paillasse comportant des bacs destinés à recevoir successivement les endoscopes en traitement et comportant chacun un détecteur capable de lire un code d'identification inscrit dans une pastille électronique collée sur chaque endoscope, toutes les étapes du processus étant gérées par un système informatique sous le contrôle d'un programme spécifique enregistrant automatiquement chaque action dans un historique non modifiable avec toutes les informations telles que nom du médecin, nom de l'opérateur, code du patient, numéro de l'endoscope, date, heure du début de l'action, durée, heure de fin de l'action, heure du retrait du bac, etc.

Sur les dessins annexés, donnés à titre d'exemple non limitatif d'une des formes de réalisation de l'objet de l'invention:
la figure 1 représente dans son ensemble, en perspective axonométrique, une installation de décontamination et de désinfection d'endoscopes,
la figure 2 est une coupe verticale suivant les flèches F1 de la figure 1 du plan de travail de la paillasse
et la figure 3 représente schématiquement, à une échelle différente, un endoscope vu de côté.

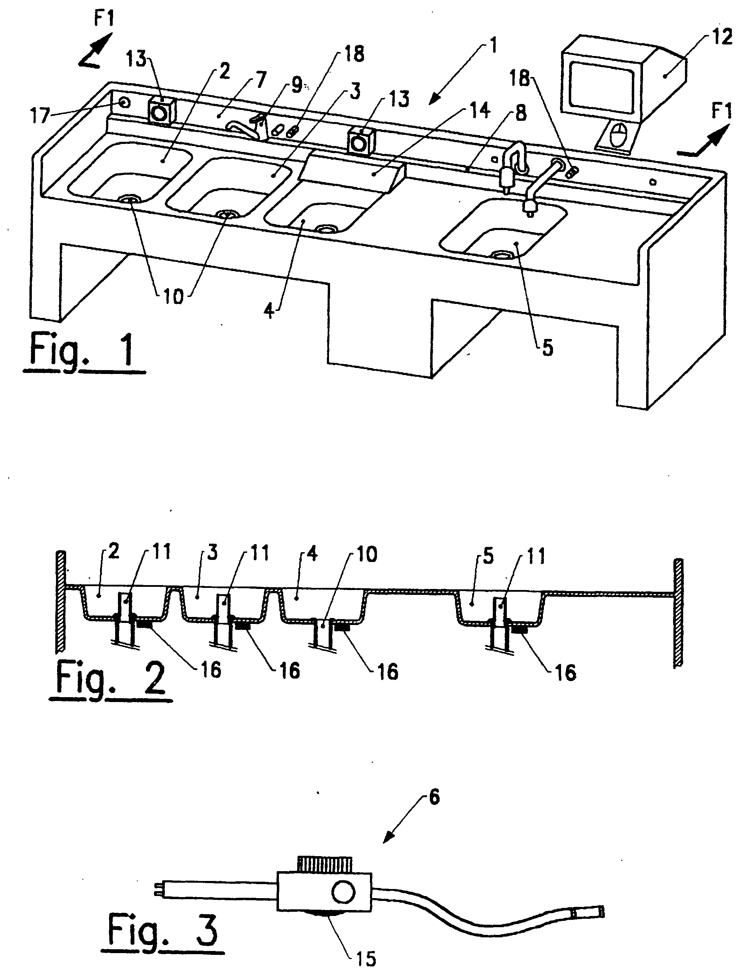

Le dispositif, figures 1 et 2, est constitué d'une paillasse 1 comportant des bacs 2 à 5 dans lesquels sont effectuées les différentes opérations, et agencée pour se conformer aux prescriptions de la circulaire de la Direction Générale de la Santé DGS/DH No. 236 du 2.4.1996 qui est le texte de référence en matière de désinfection des endoscopes. Les étapes du protocole de traitement sont effectuées dans quatre bacs différents. Une paillasse de base comporte donc au minimum quatre bacs. Il ne peut y avoir qu'un endoscope 6 par bac, mais un bac libéré peut être aussitôt utilisé pour l'endoscope suivant.

La paillasse 1 est conçue pour augmenter la qualité de la désinfection tout en simplifiant le travail de l'opérateur. Les surfaces de travail sont en produit de synthèse non poreux tel que le "Corian" (méthacrylate de méthyle à charge minérale dense d'hydroxyde d'alumine), particulièrement résistant aux agressions des produits chimiques et répondant aux normes requises dans la lutte contre les risques infectieux. Tout est conçu pour éviter la stagnation et faciliter l'accès pour le nettoyage : dosseret 7 à congé pour éviter l'encrassement du fond du plan de travail, tablette surélevée 8 pour écarter la robinetterie 9 du plan de travail, arrondi de tous les angles, placement vertical sur le dosseret 7 des boutons d'ouverture des siphons. Les équipements fixes sont en inox ou laiton chromé. Les équipements mobiles sont autoclavables.

Les bacs 2, 3, 4 et 5 de la paillasse ne comportent aucun trou, trop-plein ou trou d'aspiration, à l'exception de l'ouverture 10 du siphon, afin d'éliminer les risques de contamination par des cavités difficiles d'accès et impossibles à désinfecter de façon fiable. Le trop-plein est assuré par un manchon 11 en inox autoclavable posé verticalement sur le siphon.

Toutes les étapes sont pilotées par un ordinateur 12 et gérées par un programme informatique spécifique développé pour cette application. Chaque action est enregistrée automatiquement dans un historique non modifiable avec toutes les indications: nom du médecin, nom de l'opérateur, code du patient, numéro de l'appareil, date, heure du début de l'action, durée, heure de fin de l'action, heure du retrait du bac, information (pause, validation, interruption etc...). Cet historique peut être imprimé journellement et est archivé sur disque dur et sur disquette ou autre support.

La saisie d'un numéro de code patient propre à l'établissement permet, en cas de besoin de justificatif, d'établir un lien avec l'appareil et donc avec la désinfection précédente grâce à l'historique, tout en respectant la législation concernant l'informatique et les libertés.

Toutes les informations nécessaires au programme sont des paramètres pouvant être modifiés par une personne autorisée en fonction de l'évolution de la législation (par exemple temps de traitement) ou en fonction des besoins du service utilisateur (nouvel appareil à mémoriser, noms du personnel, nouveau protocole etc).

L'opérateur est informé du bon déroulement des opérations par un écran visualisant les bacs de la paillasse.

Le testeur manuel d'étanchéité (étape 3) est remplacé par un mini-compresseur intégré 17 et géré par le programme. La pression est tarée à la pression nécessaire pour éliminer tout risque d'endommagement de l'endoscope. L'opérateur branche un flexible immergeable sur l'endoscope et le dépose dans l'eau. Un signal sonore l'invite à rentrer des informations sur un panneau de saisie apparu à l'écran à l'aide d'une souris sans fil : nom du médecin ayant effectué l'endoscopie et nom de l'opérateur de la désinfection (à cliquer sur une liste) ainsi que le code patient (à cliquer sur l'écran numérique du panneau de saisie).

Après un délai de quelques secondes (paramètrable) l'insufflation d'air est déclenchée pendant la durée désirée pour le contrôle d'étanchéité (temps paramètrable, deux à trois minutes conseillées par les fabricants). A la fin du temps, l'opérateur doit valider le test d'étanchéité en cliquant sur le bouton apparu sur l'écran.

Pour la décontamination (étape 4), la paillasse 1 objet de l'invention utilise exclusivement des pompes péristaltiques 13 auto-amorçables. Elles aspirent le liquide dans un tuyau flexible qui, après être passé dans la tête de la pompe autour d'un rotor muni de galets qui le compriment, retourne à l'endoscope 6 permettant une circulation sans contact du liquide avec le corps de la pompe. Ce système élimine tous les problèmes de désinfection de l'intérieur des pompes basse pression. Les tuyaux flexibles ainsi que l'adaptateur pieuvre sont stérilisés en autoclave chaque jour. Cette technique augmente considérablement la fiabilité de cette étape en matière de lutte contre les risques d'affections nosocomiales.

Le risque de surpression du liquide dans les canaux est totalement éliminé soit par des galets à ressort soit par compensation électronique. Une fois l'adaptateur installé et l'endoscope déposé dans le bac, la circulation est démarrée automatiquement par le programme pour la durée prévue (paramètrable).

Le bac de rinçage 3 (étape 5) est équipé d'un tuyau flexible muni d'une pieuvre et relié à une prise d'eau indépendante tarée à 1 bar de pression. Une fois la décontamination terminée, l'endoscope non purgé est branché sur cette pieuvre. La mise en route du rinçage est automatique quelques secondes après que l'appareil ait été déposé. Dès que l'endoscope est purgé du liquide décontaminant, l'opérateur bouche la cuve avec le manchon 11 de trop-plein vertical et la remplit d'eau à l'aide d'un robinet indépendant de la prise d'eau du tuyau. L'eau propre du réseau qui coule en continu dans les canaux de l'endoscope s'ajoute à celle du bac, la faisant déborder par le trop-plein en éliminant ainsi progressivement les restes de produit décontaminant.

Le tuyau de raccordement à l'eau est équipé d'un Y dont la deuxième branche est raccordée à une prise d'air médical 18. A la fin du temps de rinçage (paramétrable, actuellement de 5 minutes), de l'air médical est soufflé automatiquement dans les canaux ce qui assure un vidage puis un séchage de l'intérieur de l'endoscope. Les résidus de détergent sont grâce à cette technique beaucoup plus sûrement éliminés qu'auparavant.

La désinfection des endoscopes 6 (étape 6) s'effectue à l'aide d'une pompe péristaltique 13. L'aspiration se fait par le haut du bac de désinfection 4. Le couvercle de ce bac, transparent pour plus de confort visuel, est un peu plus court que la longueur du bac. L'espace laissé libre à l'arrière est recouvert par une pièce de raccordement 14 du même matériau que la surface de travail, posée de biais et adossée au dosseret sur lequel se trouve l'ouverture de l'aspiration. Un bouchon d'étanchéité posé sur l'avant du couvercle permet une fois enlevé, une meilleure aspiration des vapeurs par circulation. L'étanchéité du système est assurée par un jeu de rainurages ajustés afin d'empêcher les fuites de vapeur dans l'air de la pièce pendant les périodes de non-utilisation de l'aspiration.

Un appareil de filtration placé au dessus de la paillasse 1 permet par plusieurs systèmes de traitement de recycler complètement non seulement l'air de la cuve en le débarrassant des vapeurs toxiques et des odeurs mais aussi l'air de la pièce en éliminant les poussières ainsi que les microfibres provenant des vêtements, compresses, tissus et papiers utilisés dans le service. Un filtre électrostatique lavable situé à l'avant permet de prolonger durablement la durée de vie des filtres jetables.

Le recyclage de l'air au lieu du rejet à l'extérieur est une contribution à la protection de l'environnement ainsi qu'une solution pour les locaux entourés d'autres pièces. La purification de l'air par élimination des poussières et microfibres augmente la durée de vie de filtres coûteux et facilite le nettoyage des crépines des extrémités des tuyaux d'aspiration. Elle crée les conditions de rigueur de propreté requises pour un stockage sans risque des endoscopes.

Lors du renouvellement du liquide de désinfection, la date est enregistrée en cochant la case prévue sur l'écran. Le programme informatique prend en charge le décompte du temps et des traitements et avertit l'opérateur lorsque la limite de durée de vie ou lorsque le nombre de traitements d'endoscopes est atteint.

Pour le rinçage terminal (étape 7), l'endoscope 6 est débranché de son raccord en fin de désinfection, et raccordé à l'adaptateur-pieuvre du bac de rinçage final 5. Celui-ci est équipé comme le premier bac de rinçage d'un tuyau flexible muni d'un Y se raccordant d'une part à une prise d'eau filtrée à 2 microns et d'autre part à une prise d'air médical 18. Le rinçage est déclenché automatiquement quelques secondes après que l'endoscope ait été déposé au fond du bac. Le rinçage des canaux se fait en continu. Une fois l'appareil purgé de la solution désinfectante, le bac est bouché par le manchon vertical 11 de trop-plein. Le remplissage du bac, par un robinet 9 indépendant, muni lui aussi d'un filtre à 2 microns, est déclanché par un bouton-pression situé sur le dosseret. Ce remplissage s'arrête automatiquement sans intervention de l'opérateur lorsque la hauteur du trop-plein est atteinte, grâce à une temporisation réglable. L'apport de l'eau filtrée passant dans les canaux de l'endoscope provoque l'évacuation par le trop-plein de l'eau du bain qui comporte ainsi de moins en moins de traces de dilution du produit désinfectant. A la fin du temps de rinçage (paramètrable, actuellement 5 minutes), de l'air médical est automatiquement insufflé dans l'endoscope par la deuxième branche du Y pendant plusieurs secondes (temps paramètrable également) ce qui a l'avantage non seulement de vider complètement les canaux de l'eau mais de les sécher. L'opérateur est prévenu de la fin des opérations, comme pour toutes les phases précédentes, par un signal sonore et un signal visuel sur l'écran.

Le séchage (étape 8) n'a pas lieu d'être. En effet, les canaux de l'endoscope 6 ont déjà été rigoureusement séchés par un circuit d'air à raccord étanche à la fin de l'opération précédente.

Chaque endoscope 6 est équipé d'une puce électronique 15 noyée dans matériau de synthèse, collée à l'appareil et renfermant son code. Cette puce est préférentiellement du type émetteur sans source d'énergie, le courant nécessaire à l'émission des informations à transmettre étant tiré par induction des ondes émises par des détecteurs 16 émetteurs-récepteurs disposés sous chaque bac 2 à 5. L'endoscope 6 est ainsi identifié dans chaque bac par lecture sans contact, ce qui permet d'éviter des erreurs de protocole ou de manipulations entre appareils quand plusieurs se trouvent simultanément en traitement sur la paillasse. Le programme gère les temps de traitement et le respect scrupuleux de l'ordre des opérations pour chaque endoscope. En cas d'erreur de bac, un signal sonore est émis et une information apparaît sur l'écran de l'ordinateur 12 indiquant la correction à apporter. L'opération n'est déclenchée qu'après correction.

La date et l'heure de la dernière désinfection d'un endoscope étant mémorisées par l'informatique, le système choisit le protocole de désinfection correct (complet, court ou spécial) dès que l'appareil est identifié.

L'historique des opérations effectuées peut être aussi utilisé pour des informations d'ordre statistique sur le nombre de patients traités, la fréquence d'utilisation des appareils (par exemple pour une maintenance prévisionnelle), l'évaluation des besoins en produits pour les stocks etc.

Les innovations et les améliorations apportées par le dispositif décrit sont nombreuses.
- La traçabilité grâce à la puce électronique 15 est sans conteste l'élément le plus innovant qui permet aux établissements et lieux de soin de répondre à l'obligation nouvelle de prouver le respect des normes de désinfection en cas de présomption de contamination d'un patient.
- L'utilisation de pompes péristaltiques représentent une innovation en désinfection. La suppression du contact du liquide à l'intérieur de la pompe est la solution au problème de décontamination après usage et l'annulation du risque que représentent les pompes actuelles. Le rique de surpression disparait grâce aux galets à ressorts ou au variateur de pression.
- L'utilisation du contrôle informatisé offre des garanties de rigueur du respect des procédures obligatoires de désinfection. L'enregistrement pas à pas de toutes les étapes permet un contrôle qualité par le responsable.
- Le rinçage en continu, c'est-à-dire par renouvellement constant de l'eau, n'est pas en application sur les paillasses actuelles. C'est une amélioration importante de la qualité du rinçage.
- Le travail de l'opérateur est simplifié grâce à l'automatisme de plusieurs opérations, à la détection des erreurs et aux informations constamment livrées sur l'écran.

6 - Le gain de temps sur la durée d'une désinfection et l'augmentation de la durée de vie de filtres coûteux ne sont pas négligeables.
7- L'adaptabilité du système en cas de modification des procédures grâce au paramétrage systématique des valeurs et à sa conception modulaire sont des gages de souplesse et de minimisation des coûts en cas d'évolution.

Le positionnement des divers éléments constitutifs donne à l'objet de l'invention un maximum d'effets utiles qui n'avaient pas été, à ce jour, obtenus par des dispositifs similaires.

## Revendications

1. Paillasse de décontamination d'endoscopes souples, destinée aux établissements hospitaliers en général et utilisable pour la décontamination et à la désinfection des fibroscopes, vidéo-endoscopes ou écho-endoscopes souples de tous types (gastroscopes, coloscopes, duodénoscopes, bronchoscopes, cytoscopes, etc.)
**caractérisé par** la combinaison d'une paillasse (1) comportant au moins quatre bacs (2, 3, 4, 5) destinés à recevoir successivement les endoscopes en traitement et comportant chacun un détecteur (16) capable de lire sans contact un code d'identification inscrit dans une pastille électronique (15) collée sur chaque endoscope (6), toutes les étapes du processus étant gérées par un système informatique (12) sous le contrôle d'un programme spécifique enregistrant automatiquement chaque action et toutes les informations utiles dans un historique non modifiable.

2. Paillasse de décontamination selon la revendication 1, **se caractérisant par le fait que** la pastille électronique (15) est du type émetteur sans source d'énergie, le courant nécessaire à l'émission des informations à transmettre étant tiré par induction des ondes émises par les détecteurs (16) disposés sous les bacs (2 - 5), ces détecteurs étant en fait des émetteurs-récepteurs.

3. Paillasse de décontamination selon l'une quelconque des revendications précédentes, **se caractérisant par le fait que** la décontamination et la désinfection sont toutes deux effectuées au moyen de pompes péristaltiques (13) auto-amorçables aspirant le liquide de traitement dans un tuyau flexible qui, après être passé dans la tête de la pompe autour d'un rotor muni de galets qui le compriment, retourne à l'endoscope (6) permettant une circulation sans contact du liquide avec le corps de la pompe, le risque de surpression du liquide étant éliminé soit par des galets à ressort soit par compensation électronique.

4. Paillasse de décontamination selon l'une quelconque des revendications précédentes, **se caractérisant par le fait que** les bacs (2, 3, 4, 5) de la paillasse (1) ne comportent aucun trou, trop-plein ou trou d'aspiration, à l'exception de l'ouverture du siphon (10), de manière à éliminer les risques de contamination par des cavités difficiles d'accès, le trop-plein desdits bacs étant assuré par un manchon (11) en acier inoxydable autoclavable posé verticalement sur le siphon.

5. Paillasse de décontamination selon l'une quelconque des revendications précédentes, **se caractérisant par le fait qu'**elle est équipée d'un mini-compresseur (17) intégré et géré par le programme destiné à effectuer le test d'étanchéité de l'endoscope (6).

6. Paillasse de décontamination selon l'une quelconque des revendications précédentes, **se caractérisant par le fait que** les bacs de rinçage (3, 5) sont équipés d'un tuyau flexible relié à une prise d'eau indépendante tarée à 1 bar de pression, ledit tuyau comportant une dérivation en "Y" dont la seconde branche est raccordée à une prise (18) débitant de l'air médical est soufflé automatiquement dans les canaux de l'endoscope (6) à la fin du temps de rinçage.

7. Paillasse de décontamination selon l'une quelconque des revendications précédentes, **se caractérisant par le fait que** le bac de désinfection (4) est pourvu d'un couvercle étanche avec bouchon d'étanchéité à l'avant pouvant être retiré pour permettre une meilleure aspiration des vapeurs, ledit couvercle étant un peu plus court que la longueur du bac, l'espace laissé libre à l'arrière étant recouvert par une pièce de raccordement (14) adossée au dosseret (7) sur lequel se trouve une ouverture d'aspiration.

8. Paillasse de décontamination selon l'une quelconque des revendications précédentes, **se caractérisant par le fait que** le bac de rinçage final (5) est équipé d'une prise d'eau filtrée à 2 microns, d'une prise d'air médical (18) et d'un robinet (9) indépendant, muni lui aussi d'un filtre à 2 microns destiné au remplissage du bac.

9. Paillasse de décontamination selon l'une quelconque des revendications précédentes, **se caractérisant par le fait qu'**un appareil de filtration placé au dessus de la paillasse (1) permet au moyen de plusieurs systèmes de traitement à filtres jetables de recycler complètement non seulement l'air du bac de désinfection (5) en le débarrassant des vapeurs toxiques et des odeurs mais aussi l'air de la pièce en éliminant les poussières ainsi que les microfibres provenant des vêtements, compresses, tissus et papiers utilisés dans le service, le dit appareil de filtration étant équipé d'un filtre électrostatique lavable situé à l'avant permettant de prolonger durablement la durée de vie des filtres jetables.

## Patentansprüche

1. Dekontaminationsmodule für flexible Endoskope, vorgesehen für Krankenhausanstalten im Allgemeinen und verwendbar für die Dekontamination und die Desinfektion von flexiblen Fibroskopen, Videoendoskopen oder Echoendoskopen aller Arten (Gastroskope, Koloskope, Duodenoskope, Bronchoskope, Cystoskope, usw.),
**gekennzeichnet durch** die Kombination einer Module (1) bestehend aus mindestens vier Becken (2, 3, 4, 5) zur nacheinanderfolgenden Aufnahme der zu behandelnden Endoskope, wovon jedes einen Fühler (16) aufweist, der einen Identifikationscode kontaktlos lesen kann, der auf einem auf jedes Endoskop (6) geklebten Elektronikchip (15) steht, wobei sämtliche Verfahrensschritte **durch** ein EDV-System (12) unter der Kontrolle eines spezifischen Programms gesteuert werden, das jede Aktion und alle nützlichen Informationen in einem nicht änderbaren chronologischen Überblick aufzeichnet.

2. Dekontaminationsmodule nach Anspruch 1, **gekennzeichnet dadurch, dass** der Elektronikchip (15) vom Typ Emitter ohne Energiequelle ist, wobei der zur Aussendung der zu übertragenden Informationen erforderliche Strom durch Induktion der durch die Fühler (16) ausgesendeten Wellen genommen wird; diese Fühler sind unter den Becken (2 - 5) angeordnet und sind im Grunde Sender-Empfänger.

3. Dekontaminationsmodule nach einem beliebigen der obigen Ansprüche, **gekennzeichnet dadurch, dass** die Dekontamination und die Desinfektion mittels selbstansaugender peristaltischer Pumpen (13) erfolgen, die die Behandlungsflüssigkeit in einem Schlauch ansaugen, der nach dem Passieren im Pumpenkopf um einen mit Rollen versehenen Rotor, die ihn komprimieren, zum Endoskop (6) zurückkehrt, was eine mit dem Pumpenkörper kontaktlose Zirkulation der Flüssigkeit ermöglicht und somit die Gefahr des Überdrucks der Flüssigkeit durch Federrollen oder durch elektronischer Kompensation beseitigt.

4. Dekontaminationsmodule nach einem beliebigen der obigen Ansprüche, **gekennzeichnet dadurch, dass** die Becken (2, 3, 4, 5) der Module (1) kein Loch, kein Überlaufrohr oder Saugloch aufweisen, mit Ausnahme der Siphonöffnung (10), um die Kontaminationsgefahren durch schwer zugängliche Hohlräume zu beseitigen, wobei der Überlauf der besagten Becken durch einen autoklavierenden Stutzen (11) aus rostfreiem Stahl erfolgt, der senkrecht auf den Siphon angebracht wird.

5. Dekontaminationsmodule nach einem beliebigen der obigen Ansprüche, **gekennzeichnet dadurch, dass** sie mit einem integrierten Minikompressor (17) ausgestattet ist, der durch das Programm verwaltet wird, das zur Durchführung des Dichtungstests des Endoskops (6) vorgesehen ist.

6. Dekontaminationsmodule nach einem beliebigen der obigen Ansprüche, **gekennzeichnet dadurch, dass** die Spülbecken (3, 5) mit einem Schlauch ausgestattet sind, der mit einem auf 1 Bar Druck tarierten unabhängigen Wasseranschluss verbunden ist und eine Y-Ableitung aufweist, deren zweiter Zweig mit einem Anschluss (18) verbunden ist, der medizinische Luft abgibt, die am Ende der Spülzeit automatisch in die Kanäle des Endoskops (6) geblasen wird.

7. Dekontaminationsmodule nach einem beliebigen der obigen Ansprüche, **gekennzeichnet dadurch, dass** das Desinfektionsbecken (4) mit einem dichten Deckel mit Verschlussstopfen an der Vorderseite versehen ist, der zu einer besseren Dampfabsaugung entfernt werden kann, wobei der besagte Deckel etwas kürzer als die Beckenlänge ist und der hinten frei gelassene Raum durch ein Anschlussstück (14) abgedeckt wird, das sich an der Rückwand (7) abstützt, die eine Saugöffnung aufweist.

8. Dekontaminationsmodule nach einem beliebigen der obigen Ansprüche, **gekennzeichnet dadurch, dass** das Endspülbecken (5) mit einem auf 2 Mikron gefilterten Wasseranschluss, einem medizinischen Anschluss (18) und einem unabhängigen Leitungshahn (9) ausgestattet ist, wobei letzterer mit einem 2-Mikron-Filter zum Auffüllen des Beckens versehen ist.

9. Dekontaminationsmodule nach einem beliebigen der obigen Ansprüche, **gekennzeichnet dadurch, dass** ein oberhalb der Module (1) plaziertes Filtergerät mittels mehrerer Behandlungssysteme mit Wegwerffilter es ermöglicht, nicht nur die Luft des Desinfektionsbeckens (5) komplett zu rezyklieren, indem sie von den giftigen Dämpfen und den Gerüchen befreit wird, sondern auch die Raumluft, indem der Staub sowie die Mikrofasern beseitigt werden, die von in der Abteitung verwendeten Kleidern, Kompressen, Tüchern und Papier herstammen, wobei das besagte Filtergerät an der Vorderseite mit einem waschbaren elektrostatischen Filter ausgestattet ist, mit dem die Lebensdauer der Wegwerffilter beträchtlich verlängert wird.

## Claims

1. Decontamination bench for flexible endoscopes, intended for use in hospitals in general that can be used to decontaminate and disinfect all types of flexible fibrescopes, video-endoscopes or ultra-sound endoscopes (gastroscopes, colonoscopes, duodenoscopes, bronchoscopes, cystoscopes, etc.)
**characterised by** the combination of a bench (1) containing at least four tanks (2, 3, 4, 5) intended to receive successively the endoscopes being treated and each having a detector (16) that can read the identification code on the electronic chip (15) attached to each endoscope (6) without contact, all the stages of the process are managed by an computerised system (12) controlled by a specific programme that automatically records every action and all useful information in a permanent tamper-proof archive.

2. A decontamination bench as in claim 1 **characterised by** the fact that the electronic chip (15) is an emitter without an energy source in which the current required to emit the required information is produced by induction waves emitted by the detectors (16) placed under the tanks (2, 3, 4, 5) and these detectors are in fact emitter/detectors.

3. A decontamination bench as in one of the previous claims which is **characterised by** both decontamination and disinfection being done by self-priming peristaltic pumps (13) aspiring the treatment liquid in a flexible tube which, after being passed around a rotor equipped with rollers that compress it in the pump head, returns to the endoscope (6) which provides a liquid circulation without it entering into contact with the pump chamber and where the risk of over-pressurising the liquid is eliminated either by spring mounted rollers or by an electronic compensation system.

4. A decontamination bench as in one of the previous claims which is **characterised by** having tanks (2, 3, 4, 5) on the decontamination bench (10) that have no holes, overflow or aspiration hole, except for the siphon opening (10) in such a way as to eliminate the risks of contamination from difficult to reach cavities and the overflow from these tanks is provided by an autoclavable stainless steel tube (11) placed vertically in the siphon.

5. A decontamination bench as in one of the previous claims which is **characterised by** being equipped with an integrated mini compressor (17) that is managed by the programme intended to test that the endoscope (6) is waterproof.

6. A decontamination bench as in one of the previous claims which is **characterised by** having rinsing tanks (3, 5) that are equipped with a flexible tube that is connected to an independent water intake loaded at a pressure of 1 bar where said tube has a 'Y' junction with the second branch being connected to a socket (18) connected to medical air which is automatically blown into the endoscope channels (6) at the end of rinsing.

7. A decontamination bench as in one of the previous claims which is **characterised by** having a disinfection tank (4) that has a sealed cover with a sealing stopper on the front that can be removed so that the fumes can be better aspired with said cover being a little bit shorter than the length of the tank and in which the space left free at the back is covered by a connecting piece (14) in contact with the rear (7) in which there is an aspiration hole.

8. A decontamination bench as in one of the previous claims which is **characterised by** having a final rinsing tank (5) that is equipped with a water intake that has a 2 micron filter, a medical air socket (18) and an independent tap (9) for filling the tank which is fitted with a 2 micron filter.

9. A decontamination bench as in one of the previous claims which is **characterised by** having a filtering apparatus over the bench (1) that means not only the air over the disinfection bench (5) but also the air in the room can be recycled and, by using several disposable filters, dust and micro fibres from clothing, bandages, fabrics and paper used in the department can be eliminated, said filtration apparatus being equipped with an washable electrostatic filter located at the front which extends considerable the life of the disposable filters.
